(19) Europäisches Patentamt
European Patent Office
Office européen des brevets

(11) **EP 3 087 907 A1**

(12) **EUROPEAN PATENT APPLICATION**

(43) Date of publication:
**02.11.2016 Bulletin 2016/44**

(51) Int Cl.:
*A61B 3/00* (2006.01)    *A61B 3/10* (2006.01)
*A61B 3/12* (2006.01)

(21) Application number: **16167524.4**

(22) Date of filing: **28.04.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.04.2015 JP 2015093686**

(71) Applicant: **Nidek co., Ltd.**
**Gamagori**
**Aichi (JP)**

(72) Inventors:
• **FURUUCHI, Yasuhiro**
**Gamagori, Aichi (JP)**
• **HANEBUCHI, Masaaki**
**Gamagori, Aichi (JP)**
• **SATAKE, Norimasa**
**Gamagori, Aichi (JP)**
• **TAKENO, Naoki**
**Gamagori, Aichi (JP)**

(74) Representative: **Hoefer & Partner Patentanwälte mbB**
**Pilgersheimer Straße 20**
**81543 München (DE)**

(54) **FUNDUS IMAGE PROCESSING APPARATUS, AND FUNDUS IMAGE PROCESSING METHOD**

(57)     A fundus image processing apparatus (1) includes: calculation control means (71) for acquiring a motion contrast front image (Pa1, Pa2) acquired by an OCT device (11) which is configured to acquire an OCT signal based on measurement light with which a fundus of a subject's eye (E) is scanned and reference light (S1-S3), and a fluorescent front image (FA) of the eye captured by using a contrast agent by a fundus imaging device (12) imaging the fundus of the eye (S4), and for performing positioning between the motion contrast front image and the fluorescent front image (S5).

*FIG.6*

**Description**

BACKGROUND

The present disclosure relates to a fundus image processing apparatus and a fundus image processing method for processing a fundus image of a subject's eye.

As a fundus image capturing apparatus of the related art, there is, for example, a fundus camera or a scanning laser ophthalmoscope (SLO) capturing a fundus image of a subject's eye. In such a fundus image capturing apparatus, an angiographic image of a subject's eye may be captured by using a fluorescent agent. JP-A-2011-010944 and JP-A-2015-066242 discloses a related fundus image capturing apparatus.

SUMMARY

Meanwhile, in recent years, a technique has been proposed in which motion contrast of a blood flow or the like of a subject's eye is detected by an optical coherence tomography apparatus. Consequently, a pseudo-blood vessel image is acquired.

However, a blood vessel image which is fluorescently captured by the fundus image capturing apparatus and a blood vessel image which is captured by the optical coherence tomography apparatus are different from each other in terms of type, and thus information obtained from both of the images is not effectively used.

A technical object of the present disclosure is to provide a fundus image processing apparatus and a fundus image processing program capable of effectively using information obtained from different types of images in light of the above-described problem.

In order to solve the above-described problems, the present disclosure has the following configurations.

(1) A fundus image processing apparatus comprising:

calculation control means for acquiring a motion contrast front image acquired by an OCT device which is configured to acquire an OCT signal based on measurement light with which a fundus of a subject's eye is scanned and reference light, and a fluorescent front image of the eye captured by using a contrast agent by a fundus imaging device imaging the fundus of the eye, and for performing positioning between the motion contrast front image and the fluorescent front image.

(2) The fundus image processing apparatus according to (1),
wherein the calculation control means acquires the motion contrast front image in a depth region which is common to a depth region where an imaged blood vessel is distributed in the fluorescent front image.

(3) The fundus image processing apparatus according to (1) or (2),
wherein the calculation control means detects a lesion region of the eye based on the fluorescent front image, and acquires the motion contrast front image in at least the lesion region.

(4) The fundus image processing apparatus according to (1) or (2),
wherein the calculation control means detects a lesion region of the eye based on the fluorescent front image, and performs positioning between the motion contrast front image and the fluorescent front image in a positioning region in which at least a part of the lesion region is excluded.

(5) The fundus image processing apparatus according to any one of (1) to (4),
wherein the calculation control means acquires a first motion contrast front image acquired in a first scanning region in which the fundus of the eye is scanned with the measurement light, and a second motion contrast front image acquired in a second scanning region which is different from the first scanning region, and positions the first motion contrast front image and the second motion contrast front image with the fluorescent front image.

(6) The fundus image processing apparatus according to any one of (1) to (4),
wherein the calculation control means acquires a first motion contrast front image acquired in a first scanning region in which the fundus of the eye is scanned with the measurement light, and a second motion contrast front image acquired in a second scanning region which is different from the first scanning region, and positions , with the fluorescent front image, a motion contrast combined image obtained by combining the first motion contrast front image with the second motion contrast front image.

(7) The fundus image processing apparatus according to any one of (3) to (6),
wherein the calculation control means overlaps a mark indicating a position of the lesion region on the motion contrast front image based on a result of the positioning between the motion contrast front image and the fluorescent front image.

(8) The fundus image processing apparatus according to any one of (1) to (7),
wherein the calculation control means overlaps one of the motion contrast front image and the fluorescent front image on the other the motion contrast front image and the fluorescent front image based on a result of positioning between the motion contrast front image and the fluorescent front image.

(9) The fundus image processing apparatus according to any one of (1) to (8),
wherein the calculation control means overlaps one of the motion contrast front image and the fluorescent front image on the other of the motion contrast front image and the fluorescent front image in at least the lesion region based on a result of positioning between the motion contrast front image and the fluorescent front image.

(10) The fundus image processing apparatus according to (8) or (9),
wherein the calculation control means displays the motion contrast front image and the fluorescent front image in different colors on display means.

(11) The fundus image processing apparatus according to any one of (1) to (10),
wherein the calculation control means generates a combined image of the motion contrast front image and the fluorescent front image based on a result of positioning between the motion contrast front image and the fluorescent front image.

(12) The fundus image processing apparatus according to any one of (1) to (11),
wherein the calculation control means calculates a difference between the motion contrast front image and the fluorescent front image.

(13) The fundus image processing apparatus according to any one of (1) to (12),
wherein the calculation control means matches magnifications and resolutions of the motion contrast front image and the fluorescent front image with each other, respectively.

(14) The fundus image processing apparatus according to any one of (1) to (13),
wherein the calculation control means detects image distortion information between the motion contrast front image and the fluorescent front image, and corrects distortion of at least one of the motion contrast front image and the fluorescent front image based on the distortion information.

(15) A fundus image processing method comprising:

acquiring a motion contrast front image acquired by an OCT device which is configured to acquire an OCT signal based on measurement light with which a fundus of the subject's eye is scanned and reference light;
acquiring a fluorescent front image of the subject's eye captured by using a contrast agent by a fundus imaging device imaging the fundus of the subject's eye; and
performing positioning between the motion contrast front image and the fluorescent front image.

BRIEF DESCRIPTION OF THE DRAWINGS

Fig. 1 is a schematic diagram illustrating a configuration of a fundus image processing apparatus 1.
Figs. 2A and 2B are schematic diagrams illustrating an optical system of a fundus imaging device 10.
Fig. 3 is a flowchart illustrating a control operation of the fundus image processing apparatus 1.
Figs. 4A to 4C are diagrams for explaining acquisition of motion contrast.
Fig. 5 is a diagram for explaining acquisition of a motion contrast front image.
Fig. 6 is a diagram for explaining positioning between a motion contrast front image and a fluorescent front image.
Figs. 7A and 7B are diagrams for explaining processing of a motion contrast front image and a fluorescent front image.
Fig. 8 is a diagram for explaining positioning between a motion contrast front image and a fluorescent front image.

DESCRIPTION OF ILLUSTRATIVE EMBODIMENTS

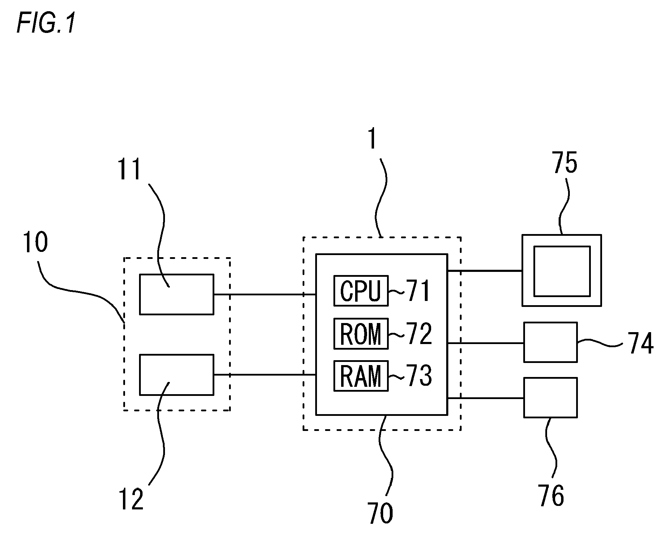

Hereinafter, the present embodiment will be described briefly with reference to the drawings. A fundus image processing apparatus (for example, a fundus image processing apparatus 1 in Fig. 1) of the present embodiment processes fundus image data captured by, for example, an imaging device (for example, an imaging device 10).

The fundus image processing apparatus mainly includes, for example, a calculation control unit (for example, a control unit 70). For example, the calculation control unit may acquire different types of fundus images (for example, a motion contrast front image and a fluorescent front image) so as to position the images. For example, the calculation control unit may acquire a fundus image captured by the imaging device. Of course, the calculation control unit may generate an image based on a signal (for example, an OCT signal or a light reception signal) acquired from the imaging device, and may acquire the generated image.

For example, the imaging device may be an OCT device (for example, an OCT device 11) or a fundus imaging device (a fundus imaging device 12).

For example, the OCT device may acquire OCT signals of measurement light with which the fundus of the subject's eye is scanned, and reference light, and may acquire a motion contrast front image based on the acquired OCT signals. A motion contrast may be, for example, information regarding motion (for example, a blood flow or a tissue change) of an object. The motion contrast front image may be, for example, a motion contrast En face image. Here, En face may be, for example, a face horizontal to a fundus face, or a two-dimensional horizontal sectional face.

For example, the fundus imaging device may capture the fundus of the subject's eye from a front direction. For example, the fundus imaging device may perform fluorescent imaging by using a fluorescent agent. For example, the fundus imaging device may acquire a fluorescent front image by fluorescently imaging the fundus of the subject's eye from the front direction. Here, the front direction may be, for example, a direction from the anterior chamber of the subject's eye toward the fundus thereof.

For example, the calculation control unit may perform positioning between a motion contrast front image and a fluorescent front image. Consequently, a blood vessel structure of the subject's eye reflected in the motion contrast front image can made to correspond to a blood vessel structure of the subject's eye reflected in the fluorescent front image.

The calculation control unit may acquire a motion contrast front image corresponding to a depth region which is common to a depth region in which an imaged blood vessel is distributed in a fluorescent front image. The depth region may be, for example, a region in a direction (for example, a depth direction or a z direction) orthogonal to the fundus face of the subject's eye.

For example, the calculation control unit may acquire information regarding in which depth region of the subject's eye an imaged blood vessel in a fluorescent front image is distributed, and may acquire a motion contrast front image generated regarding the corresponding depth region. Consequently, a blood vessel which is common to the motion contrast front image and the fluorescent front image is easily imaged, and thus positioning between both of the images is easily performed. Of course, the calculation control unit may generate a motion contrast front image regarding a corresponding depth region based on an OCT signal acquired by the OCT device.

The calculation control unit may perform positioning between a motion contrast image and a fluorescent front image in a positioning region excluding at least a part of a lesion region (for example, a leakage portion Q) of the subject's eye. For example, the calculation control unit may detect a lesion region of the subject's eye based on a fluorescent front image, and may exclude the lesion region from a positioning region. The motion contrast image and the fluorescent front image are different from each other in the way of a blood vessel structure of the lesion region being reflected therein, and thus positioning therebetween may not be performed. Therefore, the calculation control unit can favorably perform positioning between both of the images by excluding the lesion region from the positioning region.

The calculation control unit may acquire a first motion contrast front image and a second motion contrast front image. In this case, the calculation control unit may position the first motion contrast front image and the second motion contrast front image with a fluorescent front image separately. Consequently, the fluorescent front image and the motion contrast images can be correlated with each other in a plurality of regions on the fundus. The first motion contrast front image may be a motion contrast image which is acquired in a first scanning region (for example, a region A1) of measurement light on the fundus of the subject's eye. The second motion contrast front image may be a motion contrast front image which is acquired in a second scanning region (for example, a region A2) which is different from the first scanning region. Here, the scanning region may be, for example, a two-dimensional or three-dimensional region including a face horizontal to the fundus face. For example, the scanning region may be a region which is scanned in at least xy directions in a two-dimensional manner with measurement light from the OCT device.

The calculation control unit may position the first motion contrast front image and the second motion contrast front image with each other so as to acquire a motion contrast combined image. In this case, the calculation control unit may perform positioning between the motion contrast combined image and a fluorescent front image.

The calculation control unit may display a motion contrast front image and a fluorescent front image on a display unit (for example, a display unit 75) in an overlapping manner based on a result of positioning between both of the images. For example, the calculation control unit may display the motion contrast front image so as to overlap the fluorescent front image. Consequently, an examiner easily compares blood vessel structures reflected in the motion contrast front image and the fluorescent front image. Of course, the fluorescent front image may overlap the motion contrast front image.

For example, the calculation control unit may display a motion contrast front image and a fluorescent front image on the display unit in different display colors. For example, both of the images may be displayed in different colors such as red, green, blue, yellow, white, and black so that blood vessel portions of both of the images may be displayed in different colors. Consequently, an examiner can easily recognize in which image a blood vessel structure is reflected of the motion contrast front image and the fluorescent front image which overlap each other. The calculation control unit may display either one of blood vessel portions and backgrounds of both of the images in different colors.

The calculation control unit may calculate a difference between a motion contrast front image and a fluorescent front image. For example, the calculation control unit may generate a difference image by obtaining a difference between luminance of the motion contrast front image and luminance of the fluorescent front image. For example, since an abnormal blood vessel structure of a lesion region or the like which is reflected in the motion contrast front image and the fluorescent front image in different ways is left as a difference, an examiner can easily recognize the abnormal blood vessel structure of the lesion region. In a case where the difference is obtained, the calculation control unit may adjust luminances of both of the images. For example, the calculation control unit may adjust the luminance so that the luminances of blood vessel portions and backgrounds of both of the images are the same as each other. For example, the calculation control unit may binarize the luminances of both of the images so as to adjust the luminances.

The calculation control unit may match magnifications and resolutions of both images with each other when positioning a motion contrast front image and a fluorescent front image with each other. For example, the calculation control unit may easily align a position of each pixel by matching the magnifications and the resolutions of both of the images with each other. For example, the calculation control unit may match the magnifications and the resolutions of both images with each other by editing at least one of a motion contrast front image and a fluorescent front image. The calculation control unit may control the imaging device so that the magnifications and the resolutions of both of the images respectively match each other. Of course, the magnifications and the resolutions of both of the images may not necessarily match each other, and may be slightly different from each other.

The calculation control unit may correct, for example, image distortion between a motion contrast front image and a fluorescent front image. For example, the calculation control unit may detect information regarding the image distortion between the motion contrast front image and the fluorescent front image, and may correct image distortion of at least one of both of the images based on the distortion information. Consequently, the calculation control unit may easily perform positioning between both of the images. The calculation control unit may apply the distortion information of the motion contrast image to the entire motion contrast.

The calculation control unit may include a processor (for example, a CPU 71), and storage portions (for example, a ROM 72 and a storage portion 74). The processor may cause a fundus image processing program stored in the storage portion to be executed in the fundus image processing apparatus. For example, the fundus image processing program may include a calculation control step. The calculation control step may be, for example, a step of performing positioning between a motion contrast front image and a fluorescent front image.

<Example>

Hereinafter, with reference to the drawings, a fundus image processing apparatus 1 of the present example will be described. The fundus image processing apparatus 1 illustrated in Fig. 1 processes fundus image data acquired by an imaging device 10.

For example, the fundus image processing apparatus 1 includes a control unit 70. For example, the control unit 70 is realized by a general central processing unit (CPU) 71, a ROM 72, and a RAM 73. The ROM 72 stores an OCT signal processing program for processing a fundus image, various programs for controlling an operation of a device (for example, the imaging device 10 such as an OCT device 11 or a fundus imaging device 12) capturing an image of the fundus, initial values, and the like. The RAM 73 temporarily stores various information. The control unit 70 may be constituted of a plurality of control units (that is, a plurality of processors).

As illustrated in Fig. 1, the control unit 70 is electrically connected to, for example, a storage portion (for example, a nonvolatile memory) 74, an operation unit 76, and a display unit 75. The storage portion 74 is a non-transitory storage medium which can hold stored content even if the supply of power is cut. For example, a hard disk drive, a flash ROM, and an attachable and detachable USB memory may be used as the storage portion 74.

Various operation instructions are input to the operation unit 76 by an examiner. The operation unit 76 outputs a signal corresponding to an input operation instruction to the CPU 71. As the operation unit 76, for example, at least one user interface such as a mouse, a joystick, a keyboard, and a touch panel may be used.

The display unit 75 may be a display mounted in an apparatus main body, and may be a display connected to the main body. A display of a personal computer ("PC") may be used. A plurality of displays may be used. The display unit 75 may be a touch panel. If the display unit 75 is a touch panel, the display unit 75 functions as the operation unit 76. The display unit 75 displays, for example, fundus image data acquired by the imaging device 10.

The fundus image processing apparatus 1 of the present example is connected to a device (for example, the imaging device 10 such as the OCT device 11 and the fundus imaging device 12) capturing an image of the fundus. A connection method may be a wireless method, and may be a wired method. For example, the fundus image processing apparatus 1 may be configured integrally with the imaging device 10 so as to be stored in the same casing, and may be configured separately therefrom. The control unit 70 of the fundus image processing apparatus 1 may acquire fundus image data from the connected imaging device 10. Of course, the control unit 70 may not be connected to the imaging device 10. In this case, the control unit 70 may acquire fundus image data captured by the imaging device 10, via a storage medium.

<Imaging Device>

Hereinafter, the imaging device 10 will be described. The imaging device 10 images, for example, a subject's eye E. For example, the imaging device 10 includes an OCT device 11, a fundus imaging device 12, and a fixation target projection unit 300. In the present example, the imaging device 10 is connected to the control unit 70 and is thus controlled by the CPU 71. Of course, the imaging device 10 may include a CPU (not illustrated) which is different from the CPU 71 so as to perform various control for capturing fundus image data.

<OCT Device>

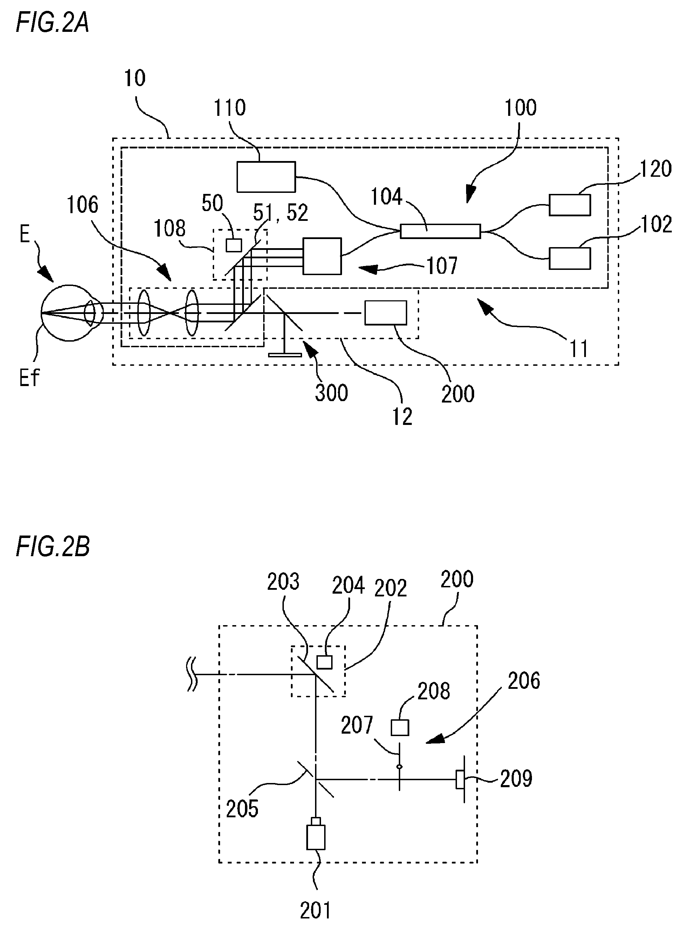

Hereinafter, with reference to Fig. 2A, a summary of the OCT device 11 will be described. For example, the OCT device 11 irradiates the subject's eye E with measurement light, and acquires an OCT signal acquired based on reflected light thereof and the measurement light. The OCT device 11 mainly includes, for example, an OCT optical system 100 and a light guide optical system 106.

<OCT Optical System>

The OCT optical system 100 irradiates the subject's eye E with measurement light. The OCT optical system 100 detects an interference state between the measurement light reflected from the subject's eye E and reference light by using a detector 120. The OCT optical system 100 includes, for example, a scanning unit (for example, an optical scanner) 108. The scanning unit 108 changes, for example, a scanning position of measurement light on the subject's eye in order to change an imaging position on the subject's eye. The CPU 71 controls an operation of the scanning unit 108 based on set scanning position information, and acquires an OCT signal based on a light reception signal from the detector 120.

The OCT optical system 100 is a so-called optical coherence tomography (OCT) optical system. The OCT optical system 100 splits light emitted from a measurement light source 102 into measurement light (sample light) and reference light by using a coupler (beam splitter) 104. The separate measurement light is guided to a measurement optical system 107, and the separate reference light is guided to a reference optical system 110. The measurement light guided to the measurement optical system 107 is guided to the fundus Ef of the eye E by the light guide optical system 106. Then, interference light obtained by combining the measurement light reflected from the subject's eye E and the reference light is received by the detector 120.

The detector 120 detects an interference state between the measurement light and the reference light. In a case of a Fourier-domain OCT, a spectral intensity of interference light is detected by the detector 120, and a depth profile (A scan signal) in a predetermined range is acquired through Fourier transform on the spectral intensity data. As the OCT optical system 100, a spectral-domain OCT (SD-OCT) optical system or a swept-source OCT (SS-OCT) optical system may be used. A time-domain OCT (TD-OCT) optical system may be used.

In a case of the SD-OCT, a low coherent light source (a wide area light source) is used as the light source 102, and the detector 120 is provided with a spectral optical system (spectrometer) which separates interference light into respective frequency components (respective wavelength components). The spectrometer is constituted of, for example, a diffraction grating and a line sensor.

In a case of the SS-OCT, a wavelength scanning type light source (wavelength variable light source) which changes emitted wavelengths temporally at a high speed is used as the light source 102, and, for example, a single light receiving element is provided as the detector 120. The light source 102 is constituted of, for example, a light source, a fiber ring resonator, and a wavelength selection filter. As the wavelength selection filter, for example, a combination of a diffraction grating and a polygon mirror, or one using Fabry-Perot etalon may be used.

Light emitted from the light source 102 is split into measurement light beams and reference light beams by the coupler 104. The measurement light beams pass through an optical fiber and are emitted into the air. The light beams are collected at the fundus Ef via the scanning unit 108 and other optical members of the light guide optical system 106. Light reflected from the fundus Ef is returned to the optical fiber along the same optical path.

The scanning unit 108 scans the fundus with measurement light in XY directions (crossing directions). The scanning unit 108 is disposed at a position substantially conjugate to the pupil. The optical scanner 108 is constituted of, for example, two galvano mirrors 51 and 52, and reflection angles thereof are arbitrarily adjusted by a driving mechanism 50. Consequently, reflection (traveling) directions of light beams emitted from the light source 102 are changed, and the fundus is scanned with the light beams in arbitrary directions. In other words, "B scan" is performed on the fundus Ef. The scanning unit 108 may have a configuration of deflecting light. For example, not only a reflective mirror (a galvano mirror, a polygon mirror, or a resonant scanner) but also an acousto-optical modulator (AOM) which changes a traveling (deflection) direction of light is used.

The reference optical system 110 generates reference light which is combined with reflected light obtained by reflection of measurement light on the fundus Ef. The reference optical system 110 may be of a Michelson type, and may be of a Mach-Zenhder type. The reference optical system 110 is constituted of, for example, a reflection optical system (for example, a reference mirror), and reflects light from the coupler 104 with the reflection optical system so that the light is returned to the coupler 104 and is thus guided to the detector 120. As another example, the reference optical system 110 is constituted of a transmission optical system (for example, an optical fiber), and transmits light from the coupler 104 without returning the light so that the light is guided to the detector 120.

The reference optical system 110 has a configuration of changing an optical path length difference between measurement light and reference light by moving an optical member on a reference optical path. For example, the reference mirror is moved in an optical axis direction. The configuration of changing an optical path length difference may be disposed on an optical path of the light guide optical system 106.

<Fundus Imaging Device>

Hereinafter, the fundus imaging device 12 will be described. The fundus imaging device 12 images the fundus Ef of the subject's eye E from the front direction (for example, an optical axis direction of measurement light). The fundus imaging device 12 mainly includes, for example, a front imaging optical system 200 and the above-described light guide optical system 106. The light guide optical system 106 of the present example is used in both of the OCT device 11 and the fundus imaging device 12, but a light guide optical system such as an objective lens may be provided in each device.

The fundus imaging device 12 is provided to obtain, for example, a front image of the fundus Ef. The fundus imaging device 12 includes, for example, a second scanning unit which scans the fundus with measurement light (infrared light) emitted from a light source in a two-dimensional manner, and a second light receiving element which receives light reflected from the fundus via a cofocal aperture which is disposed at a position substantially conjugate to the fundus, and may be a so-called scanning laser ophthalmoscope (SLO) device configuration (for example, refer to JP-A-2015-66242). A configuration of the fundus imaging device 12 may be a so-called fundus camera type configuration (refer to JP-A-2011-10944).

Fig. 2B illustrates a case where a scanning laser ophthalmoscope optical system is used as the front imaging optical system 200. In this case, the front imaging optical system 200 mainly includes, for example, a laser light source 201, a scanning unit 204, a holed mirror 205, a rotation plate unit 206, and a light receiving element 209. Of course, the front imaging optical system 200 may include other optical elements as appropriate.

For example, the laser light source 201 may emit at least laser light with a first wavelength (a wavelength of around 790 nm) and laser light with a second wavelength (a wavelength of around 490 nm). Of course, the laser light source 201 may emit only single color light.

For example, laser light from the laser light source 201 is directed toward the scanning unit 204 through an opening of the holed mirror 205 which has the opening at the center thereof. Light beams reflected by the scanning unit 204 pass through the light guide optical system 106 illustrated in Fig. 2A and are then collected at the fundus Ef of the subject's eye E. The fundus Ef is irradiated with the laser light from the laser light source 201 and thus light is emitted from the fundus Ef. For example, the laser light is scattered and reflected on the fundus Ef. As a result, the light (hereinafter also referred to as fundus reflection light) scattered and reflected on the fundus Ef is emitted through the pupil. There is a case where the laser light may excite a fluorescent substance present in the fundus Ef. Thus, there is a case where fluorescent light emitted from the fluorescent substance present in the fundus Ef may be emitted through the pupil.

The scanning unit 204 is a unit which changes (deflects laser light) a traveling direction of laser light guided from the laser light source 201 in order to scan the fundus with the laser light. In the present example, the scanning unit 204 includes a reflection mirror 203. The reflection mirror 203 may be, for example, a galvano mirror or a polygon mirror. As the scanning unit 204, a resonant scanner, or an acousto-optical modulator (AOM) which changes a traveling (deflection) direction of light may be used.

The rotation plate unit 206 selects a wavelength of light to be received by the light receiving element 209. The rotation plate unit 206 includes a rotation plate 207, a driving portion 208, and the like. For example, the rotation plate 207 has a plurality of types of barrier filters for observing fluorescent light generated from the fundus Ef. For example, the rotation plate 207 is rotated by the driving portion 208, and thus various filters are set on an optical path directed toward the light receiving element 209.

The rotation plate 207 is provided with, for example, a barrier filter for infrared fluorescent imaging and a barrier filter for visible fluorescent imaging. For example, the barrier filter for infrared fluorescent imaging is used as a barrier filter for ICGA imaging which is one kind of infrared fluorescent imaging. In addition, the indocyanine-green-angiography (ICGA) imaging is fluorescent imaging using indocyanine green as a fluorescent fundus contrast agent. For example, in a case where ICGA imaging is performed in the fundus imaging device 12 of the present example, the laser light source 201 performs irradiation with the first laser light (with a wavelength of around 790 nm) so as to perform imaging, and thus images fluorescent light with a wavelength of around 800 nm to 860 nm via the barrier filter for infrared fluorescent imaging. The ICGA imaging is mainly used for observing choroid blood vessels.

The barrier filter for visible fluorescent imaging is used for, for example, FA imaging. In addition, fluorescein-angiography (FA) imaging is fluorescent imaging using fluorescein as a fluorescent fundus contrast agent. For example, in a case where FA imaging is performed in the fundus imaging device 12 of the present example, the laser light source 201 performs irradiation with the second laser light (with a wavelength of around 490 nm), and thus images fluorescent light with a wavelength of around 510 nm to 550 nm via the barrier filter for visible fluorescent imaging. The FA imaging is mainly used for observing retinal blood vessels.

The light receiving element 209 receives light (that is, fundus reflection light during normal imaging, a fluorescent light generated from the fundus Ef during fluorescent imaging, and the like) from the fundus Ef based on the laser light from the laser light source 201. For example, during fluorescent imaging, the CPU 71 acquires a fluorescent front image of the subject's eye E based on a light reception signal from the light receiving element 209.

In a case where the fundus Ef of the subject's eye E is irradiated with laser light, light reflected or emitted from the fundus Ef based on the laser light passes through the light guide optical system 106 and the scanning unit 204 so as to be reflected by the holed mirror 205, and is then guided to the light receiving element 209 via the filter of the rotation plate 207. The pupil position of the subject's eye E may have an optical conjugate relationship with the opening of the holed mirror 205.

<Fixation Target Projection Unit>

The fixation target projection unit 300 includes an optical system for guiding a visual line direction of the eye E. The projection unit 300 has a fixation target presented to the eye E, and can guide the eye E in a plurality of directions.

For example, the fixation target projection unit 300 includes a visible light source which emits visible light, and two-dimensionally changes a target presentation position. Consequently, a visual line direction is changed, and thus an imaging part is changed. For example, if a fixation target is presented from the same direction as an imaging optical axis, a central part of the fundus is set as an imaging part. In addition, if a fixation target is presented upward with respect to the imaging optical axis, an upper part of the fundus is set as an imaging part. In other words, an imaging part is changed depending on a position of the target relative to the imaging optical axis.

The fixation target projection unit 300 may have various configurations such as a configuration in which a fixation target position is adjusted depending on turned-on positions of LEDs which are arranged in a matrix, and a configuration in which an optical scanner performs scanning with light from a light source, and a fixation target position is adjusted by controlling turning-on of the light source. The fixation target projection unit 300 may be of an internal fixation lamp type, and may be of an external fixation lamp type.

<Control Operation>

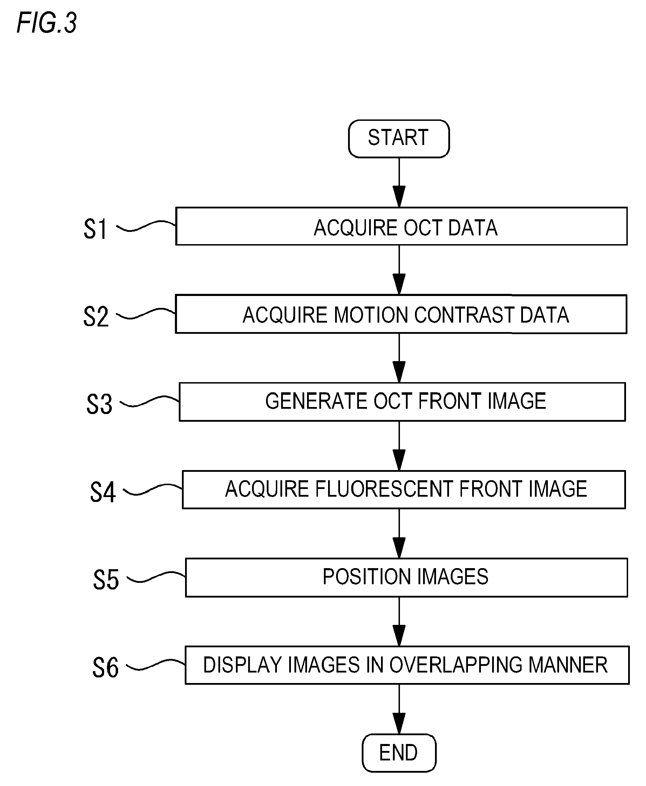

With reference to a flowchart illustrated in Fig. 3, a description will be made of a control operation in which the above-described fundus image processing apparatus 1 processes fundus image data acquired by the imaging device 10. The fundus image processing apparatus 1 of the present example positions, for example, an image obtained by the OCT device 11 with an image obtained by the fundus imaging device 12.

<Acquisition of OCT Data>

(step S1)

First, the fundus image processing apparatus 1 acquires OCT data. For example, the CPU 71 acquires an OCT signal detected by the OCT device 11, and stores data thereof in the storage portion 74 or the like. In the present example, the CPU 71 detects the OCT signal by controlling the OCT device 11.

Hereinafter, a method of the OCT device 11 detecting an OCT signal will be described. For example, the CPU 71 controls the fixation target projection unit 300 to project a fixation target onto a subject. The CPU 71 automatically performs alignment by controlling a driving unit (not illustrated) so that a measurement optical axis comes to the pupil center of the subject's eye E based on an anterior chamber observation image captured by an anterior chamber observation camera (not illustrated).

If the alignment is completed, the CPU 71 controls the OCT device 11 so as to measure the subject's eye E. The CPU 71 causes the scanning unit 108 to scan the subject's eye with measurement light, and thus acquires an OCT signal of the fundus Ef.

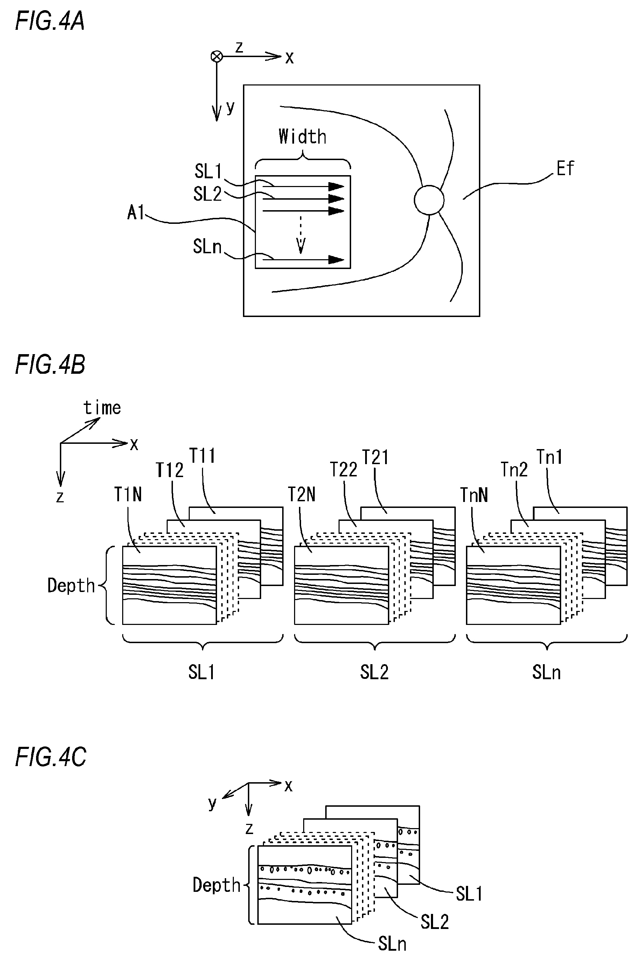

First, the CPU 71 controls the OCT device 11 to scan the fundus Ef with measurement light, and thus acquires an OCT signal. For example, as illustrated in Fig. 4A, the CPU 71 controls driving of the scanning unit 108 so that scanning with the measurement light is performed in a region A1 in which the fundus Ef is scanned with the measurement light. In Fig. 4A, a z axis direction is a direction of the optical axis of the measurement light. An x axis direction is perpendicular to the z axis and is a leftward-and-rightward direction of the subject. A y axis direction is perpendicular to the z axis and is an upward-and-downward direction of the subject.

For example, the CPU 71 performs scanning with the measurement light in the x direction along scanning lines SL1, SL2,..., and SLn in the region A1. Scanning with the measurement light in a direction (for example, the x direction) intersecting the optical axis direction of the measurement light is referred to as a "B scan". An OCT signal obtained through one B scan is referred to as an OCT signal of a single frame. The CPU 71 acquires an OCT signal detected by the detector 120 during scanning with the measurement light. The CPU 71 stores OCT data of the OCT signal acquired in the region A1, in the storage portion 74. As described above, the region A1 may be a scanning region in the xy directions, and may be a scanning region in which a plurality of scanning lines in the x direction are arranged in the y direction.

In the present example, motion contrast is acquired based on the OCT signal. The motion contrast may be, for example, information regarding a blood flow or a tissue change of the subject's eye. In a case where the motion contrast is acquired, the CPU 71 acquires at least two OCT signals which are temporally different from each other with respect to the same position on the subject's eye. For example, the CPU 71 performs a plurality of B scans in each scanning line at time intervals so as to acquire a plurality of OCT signals which are temporally different from each other. For example, the CPU 71 performs a first B scan at a certain time point, and then performs a second B scan in the same scanning line as at the first B scan after a predetermined time period elapses. The CPU 71 may acquire a plurality of OCT signals which are temporally different from each other by acquiring the OCT signals detected by the detector 120 at this time.

For example, Fig. 4B illustrates OCT data acquired in a case where a plurality of temporally different B scans are performed in the scanning lines SL1, SL2,..., and SLn. For example, Fig. 4B illustrates a case where scanning is performed at time points T11, T12,..., and T1N in the scanning line SL1, scanning is performed at time points T21, T22,..., and T2N in the scanning line SL2, and scanning is performed at time points Tn1, Tn2,..., and TnN in the scanning line SLn. As mentioned above, the CPU 71 may control the OCT device 11 to perform a plurality of temporally different B scans in each scanning line so as to acquire a plurality of temporally different OCT signals. For example, the CPU 71 acquires a plurality of temporally different OCT signals at the same position, and stores data thereof in the storage portion 74.

<Acquisition of Motion Contrast Data>

(step S2)

If the OCT data is acquired as mentioned above, the CPU 71 processes the OCT data so as to acquire motion contrast. Regarding a method of calculating the OCT data for acquiring the motion contrast, for example, there is a method of calculating an intensity difference of complex OCT signals, a method of calculating a phase difference of complex OCT signals, a method of calculating a vector difference of complex OCT signals, a method of multiplying a phase difference and a vector difference of complex OCT signals by each other, or a method (correlation mapping) using a correlation of signals. In the present example, a method of calculating a phase difference as motion contrast will be described as an example.

For example, in a case where a phase difference is calculated, the CPU 71 performs Fourier transform on a plurality of OCT signals. For example, if, among N frames, a signal at a position of an n-th frame (x,z) is indicated by a complex OCT signal $A_n(x,z)$, the CPU 71 obtains the complex OCT signal $A_n(x,z)$ through the Fourier transform. The complex OCT signal $A_n(x,z)$ includes a real number component and an imaginary number component.

The CPU 71 calculates a phase difference of at least two complex OCT signals $A(x,z)$ which are at different time points at the same position. For example, the CPU 71 calculates a phase difference by using the following Equation (1). For example, the CPU 71 may calculate a phase difference in each scanning line (refer to Fig. 4C) and may store data thereof in the storage portion 74. In addition, An in the equation indicates a signal acquired at a time point TN, and * indicates a complex conjugate.

[Equation 1]

$$\Delta\Phi_n(x, z) = \arg(A_{n+1}(x, z) \times A_n{}^*(x, z)) \qquad (1)$$

As described above, the CPU 71 acquires a motion contrast of the subject's eye based on the OCT data. As described above, a motion contrast is not limited to a phase difference, and an intensity difference, a vector difference, or the like may be acquired.

<Generation of Motion Contrast Front Image>

(step S3)

Next, the CPU 71 generates a motion contrast front image (hereinafter, abbreviated to an MC front image) based on the motion contrast data acquired in the region A1. Here, the front image may be a so-called contrast En face image. En face may be, for example, a face horizontal to a fundus face, or a two-dimensional horizontal sectional face.

As a method of generating an MC front image based on the motion contrast data, for example, there may be a method of extracting motion contrast data regarding at least some regions in the depth direction. In this case, the MC front image may be generated by using a profile of motion contrast data in at least some depth regions.

When the MC front image is generated, an example of a so-called segmentation processing method in which a region of the fundus Ef is divided in the depth direction may include a method of detecting a boundary of retinal layers of the subject's eye E from a tomographic image based on an OCT signal. For example, the CPU 71 may detect the boundary of the retinal layers of the subject's eye E by detecting an edge from an intensity image in which a luminance value is determined according to the intensity of the OCT signal. For example, the CPU 71 may divide the retinal layers of the subject's eye E into a nerve fiber layer (NFL), a ganglion cell layer (GCL), a retinal pigment epithelium (RPE), and a choroid based on the intensity image of the subject's eye E.

Since a lot of blood vessels of the retina are present in the boundary of the retinal layers, the CPU 71 may separate a region in which a lot of blood vessels are distributed based on a detection result of the boundary of the retinal layers. For example, a region within a predetermined range from the boundary of the retinal layers may be separated as a depth region in which blood vessels are distributed. Of course, the CPU 71 may separate the depth region in which blood vessels are distributed based on a distribution of blood vessels detected from a motion contrast image. For example, the CPU 71 may divide the retinal regions into a surface layer, an intermediate layer, a deep layer, and the like.

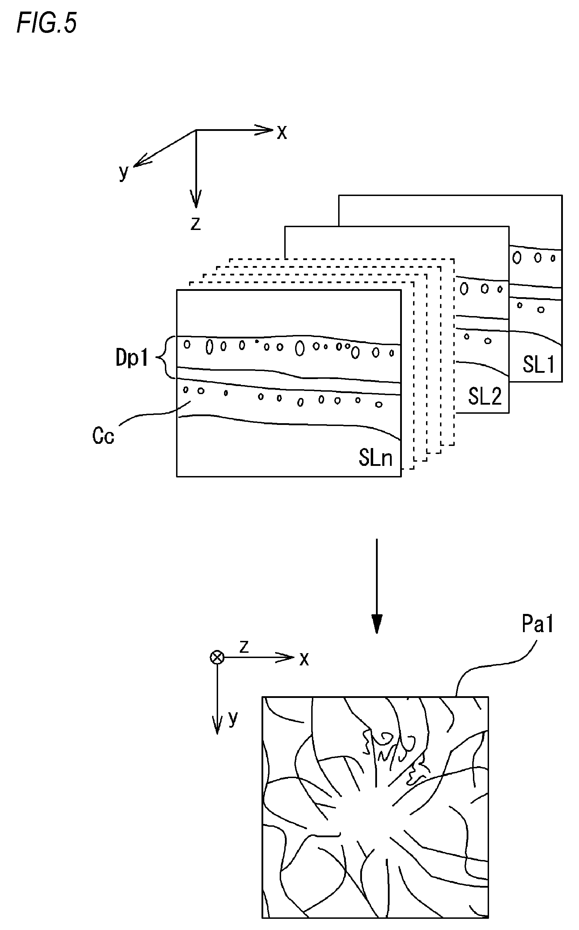

As illustrated in Fig. 5, in the present embodiment, the CPU 71 generates an MC front image Pa1 in a depth region Dp1 higher than the choroid Cc. As will be described later in detail, consequently, there is a case where positioning between an MC front image and a fluorescent front image may be easily performed.

<Acquisition of Fluorescent Front Image>

(step S4)

Successively, the fundus image processing apparatus 1 acquires fluorescent front image data. For example, the CPU 71 acquires fluorescent front image data captured by the fundus imaging device 12 and stores the data in the storage portion 74. In the present example, the CPU 71 controls the fundus imaging device 12 to perform fluorescent imaging.

For example, the CPU 71 causes the driving portion 208 to rotate the rotation plate 207 so that the barrier filter is disposed on an optical path directed toward the light receiving element 209. After alignment is completed, the examiner injects intravenously a contrast agent for fluorescent imaging into the subject's eye E, and starts fluorescent imaging by operating the operation unit 76. After the fluorescent agent is injected intravenously into the subject's eye E, the fluorescent agent is circulated to the subject's eye E, and a fluorescent image which is excited by laser light applied to the fundus appears. Here, fluorescent light which is generated on the fundus Ef by laser light emitted from the laser light source 201 is received by the light receiving element 209.

The CPU 71 acquires a light reception signal transmitted from the light receiving element 209 as fluorescent front image data. The CPU 71 may store the light reception signal from the light receiving element 209 in the storage portion 74 or the like as moving image data.

The fluorescent imaging may be performed based on a frame rate and a resolution which are set in advance.

For example, the frame rate and the resolution may be fixed, may be set by using a setting switch (not illustrated) of the operation unit 76, and may be automatically set by the CPU 71. The CPU 71 drives the scanning unit 204 at a speed at which the set frame rate and resolution are obtained.

<Positioning>

(step S5)

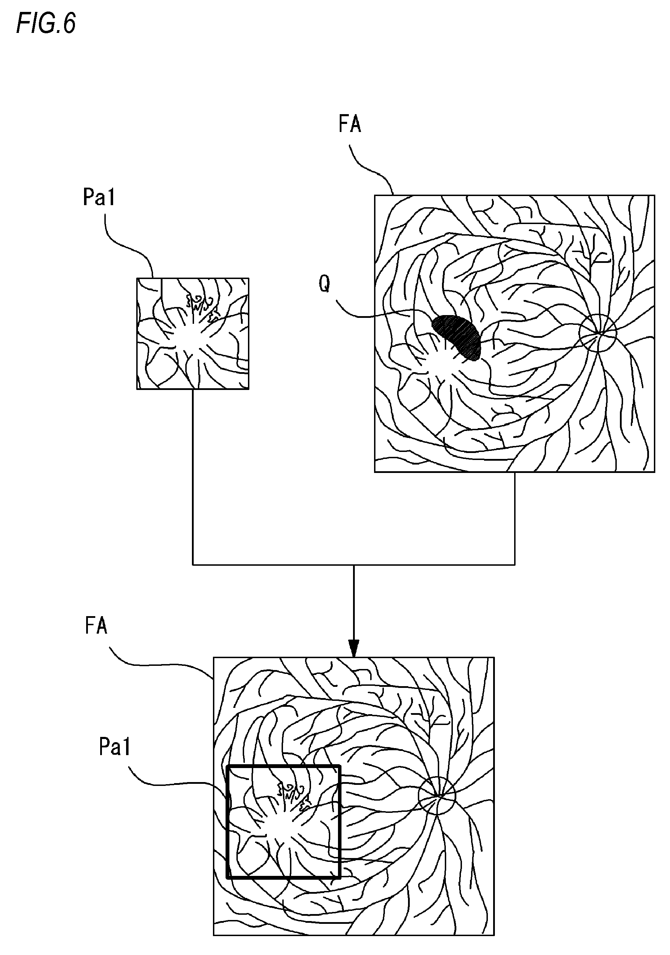

The CPU 71 performs positioning between the MC front image Pa1 acquired from the imaging device 10 and a fluorescent front image FA (refer to Fig. 6). As an image positioning method, various image processing methods such as a phase restricting correlation method, a method using Fourier transform, a method based on feature point matching, and a positioning method (for example, non-rigid registration) including affine transform and distortion correction are used.
For example, the CPU 71 may perform image positioning by positionally deviating the MC front image Pa1 and the fluorescent front image FA by one pixel so that both of the images match each other as much as possible (correlation therebetween is highest). The CPU 71 may detect positioning information such as a positional deviation direction and a positional deviation amount between both of the images. A common feature point may be extracted from the MC front image Pa1 and the fluorescent front image FA, and positioning information regarding the extracted feature point may be detected.
The MC front image Pa1 and the fluorescent front image FA are different from each other in the type of image, and thus there is a case where image positioning may be difficult. For example, if the type of blood vessel reflected in the fluorescent front image FA is different from the type of blood vessel reflected in the MC front image Pa1, structures of the blood vessel reflected in both of the images are different from each other, and thus there is a case where positioning may not normally be performed.
In this case, the CPU 71 may change a depth region for generating the MC front image Pa1 according to a blood vessel reflected in the fluorescent front image FA when generating the MC front image Pa1 based on motion contrast. For example, a blood vessel which is present in an upper layer of the choroid Cc is mainly reflected in the fluorescent front image FA acquired through FA imaging. Therefore, the CPU 71 may detect a choroid through a segmentation process, and may generate the MC front image Pa1 based on motion contrast data acquired in the depth region Dp1 located further toward the fundus surface side than the choroid (refer to Fig. 5).
As mentioned above, a depth region for generating the MC front image Pa1 is set according to the type of blood vessel reflected in the fluorescent front image FA, and thus blood vessel structures reflected in both of the images are similar to each other so that positioning is easily performed. In a case where both of the images overlap each other, resultant structures are similar to each other, and thus the examiner can easily compare both of the images.
A blood vessel of the choroid is mainly reflected in the fluorescent front image FA which is acquired through ICGA imaging. Thus, for example, the CPU 71 may acquire an MC front image in the choroid Cc so as to position the MC front image with the fluorescent front image FA.

<Display>

(step S6)

If the images are positioned with each other, the CPU 71 displays overlapping images which overlap each other based on positioning information on the display unit 75. As mentioned above, the MC front image Pa1 and the fluorescent front image FA are displayed in an overlapping manner, and thus a correspondence relationship between both of the images can be easily recognized. Therefore, the examiner can easily perform diagnosis using the MC front image Pa1 and the fluorescent front image FA.
As illustrated on the upper part of Fig. 6, in a case where a fluorescent agent leaks out of a blood vessel along with blood in a lesion portion or the like of the fundus Ef, the fluorescent front image FA is an image in which the leaking fluorescent agent is blurred white, and thus it is hard to recognize a blood vessel structure in a leakage portion Q. On the other hand, the MC front image Pa1 is an image in which a blood flow is detected, and thus it is possible to detect a blood vessel structure based on blood flow information without being influenced by leaking blood even in the leakage portion Q. In contrast, there are many cases where it is difficult to detect leaking blood in the MC front image Pa1, and thus it is hard to specify a lesion portion. Therefore, the MC front image Pa1 corresponding to the leakage portion Q where the fluorescent agent leaks is made to overlap the fluorescent front image FA, and thus the examiner can easily recognize a blood vessel structure of the leakage portion Q.
The CPU 71 may display the MC front image Pa1 in a specified region in the fluorescent front image FA. For example, the MC front image Pa1 in lesion portion which is specified in the fluorescent front image FA may be displayed on the display unit 75. Consequently, the examiner can easily recognize a blood vessel structure of a lesion portion or the like. In this case, positioning information of the MC front image Pa1 and the fluorescent front image FA is used.

Since it is also hard to recognize a blood vessel structure from the fluorescent front image FA not only in the above-described leakage, but also in lesion portions such as staining (for example, the pigment leaks due to tissue abnormality), pooling (for example, the pigment leaking out of a blood retinal barrier is pooled between tissues), and microaneurysm (for example, an aneurysm made by the pressure exerted on the arterioles), the CPU 71 may display the MC front image Pa1 in an overlapping manner so that a blood vessel structure can be easily recognized.

In a case where the fluorescent front image FA and the MC front image Pa1 overlap each other, the CPU 71 may adjust transparency of the overlapping images. For example, in a case where the MC front image Pa1 overlaps the fluorescent front image FA, the CPU 71 may adjust the transparency of the MC front image Pa1 so that the fluorescent front image FA is viewed through the MC front image Pa1. For example, the CPU 71 may adjust the transparency of the MC front image Pa1 in response to an operation on the operation unit 76. As mentioned above, both of the fluorescent front image FA and the MC front image Pa1 may be visually recognized by adjusting the transparency of overlapping images. Consequently, the examiner can easily compare the MC front image with the fluorescent front image FA.

In a case where the fluorescent front image FA and the MC front image Pa1 overlap each other, the CPU 71 may change colors of the fluorescent front image FA and the MC front image Pa1. For example, the fluorescent front image FA and the MC front image Pa1 may be displayed in different colors. For example, the fluorescent front image FA may be displayed red, and the MC front image may be displayed green. Of course, the images may be displayed by using colors (for example, blue, yellow, white, and black) other than red and green. Consequently, in a case where the fluorescent front image FA and the MC front image Pa1 overlap each other, the examiner can easily recognize on which image a blood vessel structure is based.

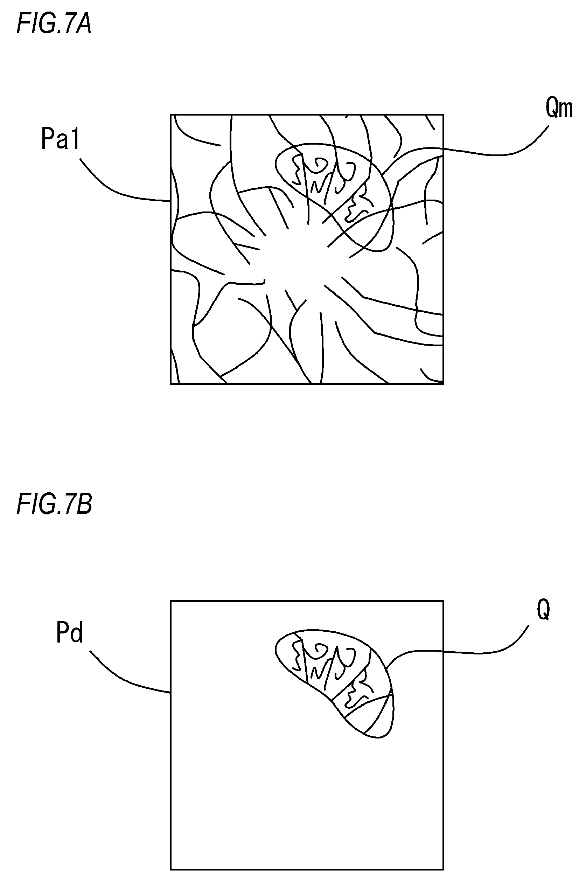

As illustrated in Fig. 7A, the CPU 71 may overlap a mark Qm indicating a position of the leakage portion Q on the MC front image Pa1 based on positioning information of the MC front image Pa1 and the fluorescent front image FA. Consequently, the examiner can recognize the position of the leakage portion Q in the MC front image Pa1.

The CPU 71 may calculate a difference between the MC front image Pa1 and the fluorescent front image FA. For example, as illustrated in Fig. 7B, the CPU 71 may generate a difference image Pd based on a difference in luminance or the like. For example, the difference image Pd is an image in which a normal blood vessel structure is removed, and only a blood vessel structure of the leakage portion Q appears. Consequently, the examiner can easily recognize a position of a lesion portion, a blood vessel structure, and the like. When calculating a difference between the MC front image Pa1 and the fluorescent front image FA, the CPU 71 may adjust luminances of both of the images so that a normal blood vessel structure is smoothly canceled out. For example, the CPU 71 may adjust the luminances of both of the images through binarization so that a binarized difference between both of the images is calculated.

As described above, the MC front image Pa1 and the fluorescent front image FA are different from each other in the way of a lesion portion (for example, the leakage portion Q) being reflected therein. In this case, a correlation between the images may not be taken in the lesion portion, and this may cause difficulties in positioning. Therefore, the CPU 71 may perform positioning between the MC front image and the fluorescent front image FA in a region other than the lesion portion. For example, the CPU 71 may specify a lesion portion through analysis or the like of a luminance value, and may perform positioning between the fluorescent front image FA and the MC front image Pa1 in a positioning region other than the specified lesion portion. Consequently, it is possible to perform positioning between the MC front image Pa1 and the fluorescent front image FA without being influenced by a difference in the way of a lesion portion being reflected therein.

Examples of methods of specifying a lesion portion may include a method of comparing a fundus front image obtained through normal imaging with the fluorescent front image FA, and a method of comparing a plurality of fluorescent front images FA captured at different time points with each other. For example, the fluorescent front image FA captured right after a fluorescent agent is intravenously injected may be compared with the fluorescent front image FA captured after a while from the intravenous injection of the fluorescent agent. In this case, a lesion portion may be detected based on a change in images before and after the fluorescent agent leaks out of a blood vessel. Of course, a lesion portion may be specified based on an operation on the operation unit 76.

In image positioning, magnifications and resolutions of the MC front image Pa1 and the fluorescent front image FA may be respectively set to be equivalent to each other. For example, the CPU 71 may control the OCT device 11 and the fundus imaging device 12 to capture both of the images so that imaging magnifications and resolutions of the MC front image Pa1 and the fluorescent front image FA are respectively equivalent to each other. For example, the CPU 71 may control driving of the scanning units (for example, the scanning units 108 and 204) or the like of the imaging device 10 so that the imaging magnifications and the resolutions are respectively equivalent to each other. The imaging magnifications and the resolutions are respectively equivalent to each other, and thus positioning is easily performed through image processing. Of course, the resolutions may not be the same as each other, and may be similar to each other.

In a case where the CPU 71 does not control the OCT device 11 and the fundus imaging device 12, that is, the CPU 71 acquires fundus image data by using the external imaging device 10, the CPU 71 may edit at least one fundus image of the MC front image Pa1 and the fluorescent front image FA. For example, the CPU 71 may change the resolution of at least one fundus image of the MC front image and the fluorescent front image FA so that the imaging magnifications and the resolutions of the MC front image Pa1 and the fluorescent front image FA are respectively equivalent to each other. For example, the CPU 71 may acquire the imaging magnifications and the resolutions of the MC front image Pa1 and the fluorescent front image FA from the OCT device 11 and the fundus imaging device 12. For example, the CPU 71 may reduce the resolution of an image with a larger resolution of the MC front image Pa1 and the fluorescent front image FA for adjustment to an image with a smaller resolution so that the resolutions of both of the images are equivalent to each other.

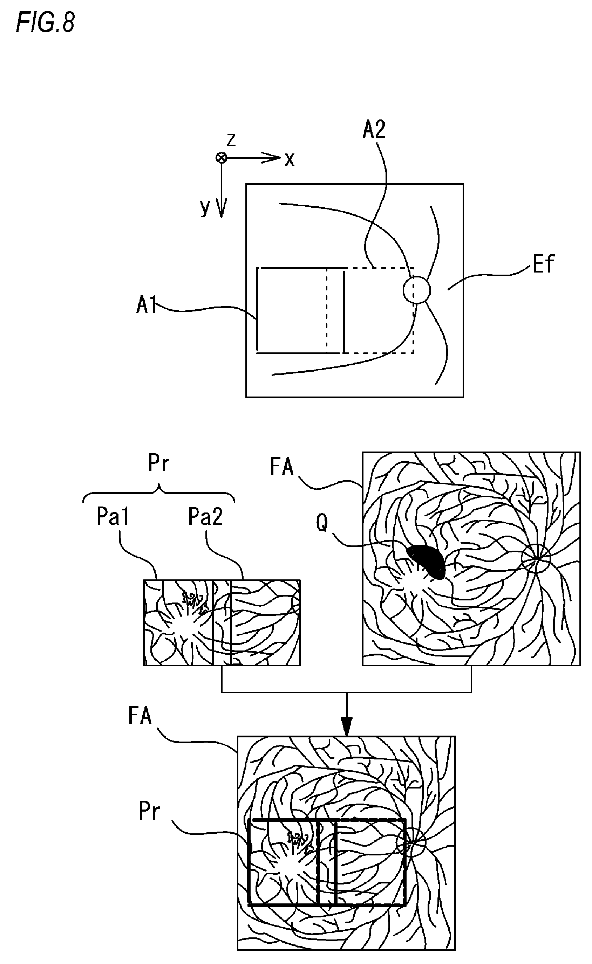

In a case where the fluorescent front image FA and the MC front image overlap each other, a plurality of MC front images may overlap the fluorescent front image FA. For example, as illustrated in Fig. 8, the CPU 71 acquires motion contrasts in a region A1 and a region A2, and may generate MC front images Pa1 and Pa2 in the region A1 and the region A2, respectively. The CPU 71 may overlap the MC front image Pa1 and the MC front image Pa2 on a single fluorescent front image FA. The region A1 and the region A2 may be regions on the fundus of which at least parts are different from each other, and may be totally different regions.

In a case where a plurality of MC front images are positioned with a single fluorescent front image FA, each of the plurality of MC front images Pa1 and Pa2 may be positioned with the fluorescent front image FA, and a combined image Pr obtained by positioning the plurality of MC front images Pa1 and Pa2 with each other in advance may be positioned with the fluorescent front image FA.

Regarding an order of acquiring an MC front image and a fluorescent front image, either thereof may be acquired first, and both of the images may be simultaneously acquired. For example, a fluorescent front image may be first captured by the fundus imaging device 12, and then an OCT signal may be acquired by the OCT device 11. In this case, the CPU 71 may scan a region (for example, a lesion portion) specified in the fluorescent front image with measurement light from the OCT device 11 so as to acquire motion contrast. Consequently, it is possible to accurately acquire an MC front image of a lesion portion or the like.

<Distortion Correction>

The CPU 71 may correct, for example, distortion of a front image. For example, it takes a long time to measure motion contrast, and thus there is a case where an MC front image may be distorted. As mentioned above, in a case where an MC front image is distorted relative to the fluorescent front image FA, feature regions (for example, a blood vessel portion) of both of the images do not match each other, and thus positioning is difficult. In such a case, the CPU 71 may perform a positioning process (for example, non-rigid registration) including distortion correction on the MC front image and the fluorescent front image FA. Consequently, even in a case where distortion occurs in at least a part of the MC front image or the like, it is possible to appropriately perform positioning between the MC front image and the fluorescent front image. The CPU 71 may develop a correction amount in which the distortion correction is performed on the MC front image into three-dimensional motion contrast data so as to correct distortion of the entire motion contrast data.

An imaging condition for each image may be used for positioning between the MC front image and the fluorescent front image FA. For example, imaging position information may be used for positioning between the images. For example, the CPU 71 may perform positioning in a state in which an imaging position of each front image is specified to some extent by using the imaging position information. In this case, the CPU 71 may use, as the imaging position information, a scanning position of measurement light when each front image is acquired, a fixation position of a fixation target presented to the subject's eye E, and the like. As mentioned above, the CPU 71 may increase a processing speed for image positioning by using the imaging position information of each front image.

The CPU 71 may use other types of image data for positioning between an MC front image and a fluorescent front image. For example, the CPU 71 may acquire an image captured by an imaging optical system which is different from the OCT device 11 and the fundus imaging device 12. For example, other types of images may include an infrared front image. For example, the CPU 71 may detect a positional deviation amount between a first infrared front image obtained when an MC front image is captured and a second infrared front image obtained when a fluorescent front image is captured, and may perform positioning between the MC front image and the fluorescent front image based on the detected positional deviation amount. For example, the CPU 71 may position an MC front image and a fluorescent front image with the same infrared front image, and may position the MC front image and the fluorescent front image with each other based on acquired positioning information.

The fluorescent front image used to be positioned with the MC front image may be, for example, a fluorescent front image captured right after a contrast agent is injected, and may be a fluorescent front image captured in a state in which the contrast agent spreads into blood vessels after some time elapses.

Description of Reference Numerals and Signs

| | |
|---|---|
| 10 | IMAGING DEVICE |
| 11 | OCT DEVICE |
| 12 | FUNDUS IMAGING DEVICE |
| 70 | CONTROL UNIT |
| 71 | CPU |
| 72 | ROM |
| 73 | RAM |
| 74 | STORAGE PORTION |
| 75 | MONITOR |
| 76 | OPERATION UNIT |
| 100 | OCT OPTICAL SYSTEM |
| 108 | SCANNING UNIT |
| 200 | FRONT IMAGING OPTICAL SYSTEM |
| 300 | FIXATION TARGET PROJECTION UNIT |

**Claims**

1. A fundus image processing apparatus comprising:

   calculation control means for acquiring a motion contrast front image acquired by an OCT device which is configured to acquire an OCT signal based on measurement light with which a fundus of a subject's eye is scanned and reference light, and a fluorescent front image of the eye captured by using a contrast agent by a fundus imaging device imaging the fundus of the eye, and for performing positioning between the motion contrast front image and the fluorescent front image.

2. The fundus image processing apparatus according to claim 1,
   wherein the calculation control means acquires the motion contrast front image in a depth region which is common to a depth region where an imaged blood vessel is distributed in the fluorescent front image.

3. The fundus image processing apparatus according to claim 1 or 2,
   wherein the calculation control means detects a lesion region of the eye based on the fluorescent front image, and acquires the motion contrast front image in at least the lesion region.

4. The fundus image processing apparatus according to claim 1 or 2,
   wherein the calculation control means detects a lesion region of the eye based on the fluorescent front image, and performs positioning between the motion contrast front image and the fluorescent front image in a positioning region in which at least a part of the lesion region is excluded.

5. The fundus image processing apparatus according to any one of claims 1 to 4,
   wherein the calculation control means acquires a first motion contrast front image acquired in a first scanning region in which the fundus of the eye is scanned with the measurement light, and a second motion contrast front image acquired in a second scanning region which is different from the first scanning region, and positions the first motion contrast front image and the second motion contrast front image with the fluorescent front image.

6. The fundus image processing apparatus according to any one of claims 1 to 4,
   wherein the calculation control means acquires a first motion contrast front image acquired in a first scanning region in which the fundus of the eye is scanned with the measurement light, and a second motion contrast front image acquired in a second scanning region which is different from the first scanning region, and positions , with the fluorescent front image, a motion contrast combined image obtained by combining the first motion contrast front image with the second motion contrast front image.

7. The fundus image processing apparatus according to any one of claims 3 to 6,
   wherein the calculation control means overlaps a mark indicating a position of the lesion region on the motion contrast front image based on a result of the positioning between the motion contrast front image and the fluorescent front image.

8. The fundus image processing apparatus according to any one of claims 1 to 7,
wherein the calculation control means overlaps one of the motion contrast front image and the fluorescent front image on the other the motion contrast front image and the fluorescent front image based on a result of positioning between the motion contrast front image and the fluorescent front image.

9. The fundus image processing apparatus according to any one of claims 1 to 8,
wherein the calculation control means overlaps one of the motion contrast front image and the fluorescent front image on the other of the motion contrast front image and the fluorescent front image in at least the lesion region based on a result of positioning between the motion contrast front image and the fluorescent front image.

10. The fundus image processing apparatus according to claim 8 or 9,
wherein the calculation control means displays the motion contrast front image and the fluorescent front image in different colors on display means.

11. The fundus image processing apparatus according to any one of claims 1 to 10,
wherein the calculation control means generates a combined image of the motion contrast front image and the fluorescent front image based on a result of positioning between the motion contrast front image and the fluorescent front image.

12. The fundus image processing apparatus according to any one of claims 1 to 11,
wherein the calculation control means calculates a difference between the motion contrast front image and the fluorescent front image.

13. The fundus image processing apparatus according to any one of claims 1 to 12,
wherein the calculation control means matches magnifications and resolutions of the motion contrast front image and the fluorescent front image with each other, respectively.

14. The fundus image processing apparatus according to any one of claims 1 to 13,
wherein the calculation control means detects image distortion information between the motion contrast front image and the fluorescent front image, and corrects distortion of at least one of the motion contrast front image and the fluorescent front image based on the distortion information.

15. A fundus image processing method comprising:

acquiring a motion contrast front image acquired by an OCT device which is configured to acquire an OCT signal based on measurement light with which a fundus of the subject's eye is scanned and reference light;
acquiring a fluorescent front image of the subject's eye captured by using a contrast agent by a fundus imaging device imaging the fundus of the subject's eye; and
performing positioning between the motion contrast front image and the fluorescent front image.

FIG.1

*FIG.2A*

*FIG.2B*

FIG.3

```
                    ┌──────────┐
                    │  START   │
                    └────┬─────┘
                         ▼
S1 ──────  ┌─────────────────────────────────────┐
           │          ACQUIRE OCT DATA            │
           └──────────────────┬──────────────────┘
                              ▼
S2 ──────  ┌─────────────────────────────────────┐
           │     ACQUIRE MOTION CONTRAST DATA     │
           └──────────────────┬──────────────────┘
                              ▼
S3 ──────  ┌─────────────────────────────────────┐
           │       GENERATE OCT FRONT IMAGE       │
           └──────────────────┬──────────────────┘
                              ▼
S4 ──────  ┌─────────────────────────────────────┐
           │    ACQUIRE FLUORESCENT FRONT IMAGE   │
           └──────────────────┬──────────────────┘
                              ▼
S5 ──────  ┌─────────────────────────────────────┐
           │            POSITION IMAGES           │
           └──────────────────┬──────────────────┘
                              ▼
S6 ──────  ┌─────────────────────────────────────┐
           │  DISPLAY IMAGES IN OVERLAPPING MANNER │
           └──────────────────┬──────────────────┘
                              ▼
                         ┌─────────┐
                         │   END   │
                         └─────────┘
```

FIG.4A

FIG.4B

FIG.4C

*FIG.5*

FIG.6

*FIG.7A*

Pa1

Qm

*FIG.7B*

Pd

Q

FIG.8

Europäisches
Patentamt

European
Patent Office

Office européen
des brevets

# EUROPEAN SEARCH REPORT

Application Number

EP 16 16 7524

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JEFF FINGLER ET AL: "Volumetric microvascular imaging of human retina using optical coherence tomography with a novel motion contrast technique", OPTICS EXPRESS, vol. 17, no. 24, 19 November 2009 (2009-11-19), pages 22190-22200, XP055291503, DOI: 10.1364/OE.17.022190 | 1,2,5,6, 8,9, 11-15 | INV. A61B3/00 A61B3/10 A61B3/12 |
| Y | * Sections 3 and 4 * <br> * figures 1, 3-5, 7 * | 3,4,7 | |
| X | DAE YU KIM ET AL: "In vivo volumetric imaging of human retinal circulation with phase-variance optical coherence tomography", BIOMEDICAL OPTICS EXPRESS, vol. 2, no. 6, 11 May 2011 (2011-05-11), pages 1504-1513, XP055291698, United States ISSN: 2156-7085, DOI: 10.1364/BOE.2.001504 | 1,10,15 | |
| Y | * page 1512, last paragraph - page 1513, paragraph 1 * <br> * figure 8 * | 3,4,7 | TECHNICAL FIELDS SEARCHED (IPC) <br><br> A61B |

-/--

The present search report has been drawn up for all claims

| Place of search | Date of completion of the search | Examiner |
|---|---|---|
| Munich | 28 July 2016 | Gärtner, Andreas |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

# EUROPEAN SEARCH REPORT

Application Number

EP 16 16 7524

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PODDAR RAJU ET AL: "imaging of human vasculature in the chorioretinal complex using phase-variance contrast method with phase-stabilizedswept-source optical coherence tomography", JOURNAL OF BIOMEDICAL OPTICS, S P I E - INTERNATIONAL SOCIETY FOR OPTICAL ENGINEERING, US, vol. 19, no. 12, 1 December 2014 (2014-12-01), page 126010, XP060047175, ISSN: 1083-3668, DOI: 10.1117/1.JBO.19.12.126010 [retrieved on 2014-12-17] | 1,2,15 | |
| Y | * page 8, right-hand column, last paragraph - page 9, left-hand column, paragraph 1 * * page 11, left-hand column, paragraph 3 - paragraph 5 * * figures 7, 11 * | 3,4,7 | |
| X | DANIEL M. SCHWARTZ ET AL: "Phase-Variance Optical Coherence Tomography", OPHTHALMOLOGY., vol. 121, no. 1, 1 January 2014 (2014-01-01), pages 180-187, XP055291677, US ISSN: 0161-6420, DOI: 10.1016/j.ophtha.2013.09.002 | 1,15 | |
| Y | * page 184 * * figure 4 * | 3,4,7 | |
| Y | US 2014/276025 A1 (DURBIN MARY K [US] ET AL) 18 September 2014 (2014-09-18) * paragraphs [0053] - [0061] * * paragraphs [0067] - [0074] * * paragraph [0094] * * figures 4, 5 * | 3,4,7 | |

TECHNICAL FIELDS
SEARCHED (IPC)

The present search report has been drawn up for all claims

| Place of search | Date of completion of the search | Examiner |
|---|---|---|
| Munich | 28 July 2016 | Gärtner, Andreas |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 16 16 7524

This annex lists the patent family members relating to the patent documents cited in the above-mentioned European search report.
The members are as contained in the European Patent Office EDP file on
The European Patent Office is in no way liable for these particulars which are merely given for the purpose of information.

28-07-2016

| Patent document cited in search report | Publication date | Patent family member(s) | Publication date |
|---|---|---|---|
| US 2014276025 A1 | 18-09-2014 | EP 2967317 A2<br>JP 2016509914 A<br>US 2014276025 A1<br>WO 2014140258 A2 | 20-01-2016<br>04-04-2016<br>18-09-2014<br>18-09-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011010944 A

- JP 2015066242 A